(19) Europäisches Patentamt
European Patent Office
Office européen des brevets

(11) Veröffentlichungsnummer: **0 307 814 B1**

# (12) EUROPÄISCHE PATENTSCHRIFT

(45) Veröffentlichungstag der Patentschrift: **08.04.92**

(21) Anmeldenummer: **88114755.7**

(22) Anmeldetag: **09.09.88**

(51) Int. Cl.⁵: **C07D 487/14**, A61K 31/505, A61K 31/55, //(C07D487/14, 239:00,235:00,209:00), (C07D487/14,239:00,239:00, 209:00),(C07D487/14,243:00, 209:00)

## (54) Tetracyclische Chinazolinderivate, Herstellung und Verwendung.

(30) Priorität: **12.09.87 DE 3730718**

(43) Veröffentlichungstag der Anmeldung:
**22.03.89 Patentblatt 89/12**

(45) Bekanntmachung des Hinweises auf die Patenterteilung:
**08.04.92 Patentblatt 92/15**

(84) Benannte Vertragsstaaten:
**AT BE CH DE ES FR GB IT LI NL**

(56) Entgegenhaltungen:
**EP-A- 0 046 446**
**US-A- 3 441 566**

(73) Patentinhaber: **BASF Aktiengesellschaft**
**Carl-Bosch-Strasse 38**
**W-6700 Ludwigshafen(DE)**

(72) Erfinder: **Franke, Albrecht, Dr.**
**11 Mandelring**
**W-6706 Wachenheim(DE)**
Erfinder: **Ostersehlt, Bernd, Dr.**
**Birkenstrasse 18**
**W-6701 Maxdorf(DE)**
Erfinder: **Schlecker, Rainer, Dr.**
**Suedring 8**
**W-6719 Bissersheim(DE)**
Erfinder: **Rendenbach, Beatrice, Dr.**
**Kapellenstrasse 8**
**W-6701 Waldsee(DE)**
Erfinder: **Von Philipsborn, Gerda, Dr.**
**35 Naechstenbacher Weg**
**W-6940 Weinheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 307 814 B1

Rank Xerox (UK) Business Services

**Beschreibung**

Gegenstand der Erfindung sind neue tetracyclische Chinazolinderivate, die wertvolle therapeutische, insbesondere antiarrhythmische Eigenschaften aufweisen, Verfahren zu deren Herstellung sowie deren Verwendung bei der Bekämpfung von Krankheiten.

Es ist bekannt, daß substituierte Chinazoline blutdrucksenkende und bronchodilatatorische Eigenschaften besitzen (US-A-3.441.566, DE-A-2.162.590). In der EP-A-0.046.446 werden polyazaheterocyclische Verbindungen beschrieben, die diuretische, natriuretische, hypoglykämische, antiphiogistische und antinociceptive Eigenschaften besitzen.

Es wurde nun gefunden, daß tetracyclische Chinazolinderivate der Formel I

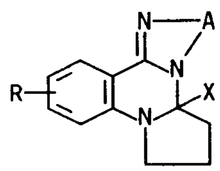

I,

worin

A eine gegebenenfalls $C_{1-4}$-alkylsubstituierte $C_{2-4}$-Alkylengruppe, worin 2 benachbarte Methylengruppen zusätzlich über eine gegebenenfalls $C_{1-4}$-alkylsubstituierte $C_3$-$C_5$-Alkylengruppe zu einem Ring geschlossen sein können.

X einen gegebenenfalls durch Halogen, Nitro, Amino, $C_{1-4}$-Alkylamino, Sulfonylamino, $C_{1-4}$-Acylamino, Hydroxy, $C_{1-4}$-Alkoxy, $-O(CH_2)_{2-4}-NR^1R^2$ (mit $R^1$ und $R^2$ in der Bedeutung von Wasserstoff oder $C_{1-4}$-Alkyl),$C_{1-4}$-Alkyl oder eine $C_{1-4}$-Alkylsulfonsäuregruppe substituierten Phenyl- oder Naphthylring oder einen gegebenenfalls durch Chlor, Fluor, Methyl, Ethyl, Methoxy, Ethoxy, Nitro oder Hydroxy substituierten Thiophenring und

R Wasserstoff, Halogen oder $C_{1-4}$-Alkoxy

bedeuten, sowie deren Salze mit physiologisch verträglichen Säuren überraschenderweise Antiarrhythmica der Klasse III darstellen. In der Formel I bedeutet A vorzugsweise eine gegebenenfalls durch ein oder zwei $C_{1-4}$-Alkylreste substituierte $C_{2-3}$-Alkylengruppe und insbesondere einen durch ein oder zwei Methylreste substituierten $C_{2-3}$-Alkylenrest, X vorzugsweise einen durch Wasserstoff, Chlor, Fluor, Methyl, Ethyl, Methoxy, Ethoxy, Nitro oder Hydroxy substituierten Phenyl- oder Thiophenring und R vorzugsweise Ethoxy und insbesondere Wasserstoff, Fluor, Chlor und Methoxy.

Die Verbindungen der Formel I lassen sich herstellen, indem man

a) eine Verbindung der Formel II

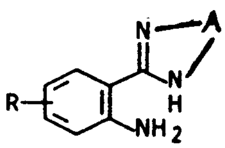

II,

in der A und R die angegebenen Bedeutungen haben, mit einem ω-Halogenketon der Formel III

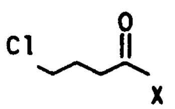

III,

in dem X die angegebene Bedeutung hat, umsetzt oder

b) eine Verbindung der Formel IV

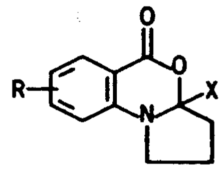

IV,

worin R und X die angegebenen Bedeutungen besitzen, mit einem Diamin der Formel V

$H_2N\text{-}A\text{-}NH_2$ V ,

in der A die angegebene Bedeutung hat, umsetzt
und die so erhaltenen Verbindungen der Formel I, falls X eine durch eine Hydroxygruppe substituierte Phenyl- oder Naphthylgruppe bedeutet, gegebenenfalls mit einem Alkylhalogenid der Formel VI

$Hal\text{-}(CH_2)_{2-4}\text{-}NR^1R^2$ VI ,

worin Hal ein Halogenatom bedeutet und $R^1$ und $R^2$ die angegebenen Bedeutungen haben, oder einem $C_{1-4}$-Alkylsulfonylchlorid umsetzt und anschließend die erhaltenen Verbindungen gegebenenfalls in ihre Salze mit physiologisch verträglichen Säuren überführt.

Im Falle des Verfahrens a) erfolgen die Umsetzungen vorzugsweise in Gegenwart eines Verdünnungs- oder Lösungsmittels, beispielsweise eines niederen Alkohols, und zweckmäßigerweise bei Temperaturen zwischen 25 ° C und der Siedetemperatur des verwendeten Lösungsmittels. Zweckmäßigerweise werden die Umsetzungen unter Zusatz einer Mineralsäure, z. B. HCl und gegebenenfalls in Anwesenheit katalytischer Mengen NaI durchgeführt.

Bevorzugtes Lösungsmittel bei der Umsetzung der Verbindungen II und III ist Ethanol, wobei die Reaktion bevorzugt bei der Siedetemperatur des Lösungsmittels durchgeführt wird.

Die vollständige Umsetzung hängt von den Edukten ab und ist im allgemeinen innerhalb von 8 bis 60 Stunden beendet. Gegebenenfalls wird zur Vervollständigung der Reaktion das Lösungsmittel entfernt und der Rückstand 2 bis 120 Stunden auf Temperaturen von 90 bis 160 ° C, vorzugsweise auf 110 bis 130 ° C, zweckmäßigerweise unter $N_2$-Atmosphäre erhitzt. Das Reaktionsprodukt kann in an sich üblicher Weise gewonnen werden, z. B. durch Filtration, Abdestillieren des Verdünnungs-oder Lösungsmittels aus dem Reaktionsgemisch oder Extraktion. Eine Reinigung der erhaltenen Verbindung erfolgt in üblicher Weise, beispielsweise durch Umkristallisieren aus einem Lösungsmittel oder Überführen in eine Säureadditionsverbindung.

Die Ausgangsverbindungen der allgemeinen Formel II sind zum Teil bekannt oder können nach literaturbekannten Methoden, wie sie beispielsweise in Houben-Weyl, Methoden der organischen Chemie, Bd. 11/2, S. 38 ff., G. Thieme Verlag, Stuttgart 1958 beschrieben sind, durch Umsetzung eines entsprechenden o-Aminonitrils mit einem Diamin der allgemeinen Formel V, vorzugsweise unter Zusatz von katalytischen Mengen Ammoniumsulfid und Schwefel, hergestellt werden.

Die ω-Halogenketone der allgemeinen Formel III sind literaturbekannt und können durch Friedel-Crafts-Acylierung entsprechend substituierter Aromaten mit ω-Chlorbuttersäurechlorid nach Methoden wie sie beispielsweise in Pharmazie 35, 140 (1980) oder Industrie Chimique Belge, 9, 1073 (1960) beschrieben sind, dargestellt werden. Verbindungen der allgemeinen Formel III werden auch durch nucleophile Ringöffnung entsprechend substituierter Cyclopropylphenylketone mit HCl entsprechend der in Journal of Labelled Compounds and Radiopharmaceuticals 21, 533 (1984) beschriebenen Methode erhalten.

Die Umsetzung von Verbindungen der allgemeinen Formel IV mit einem Diamin V nach Verfahren b) kann bei Raumtemperatur oder höheren Temperaturen, zweckmäßig bei Temperaturen zwischen 60 und 100 ° C durchgeführt werden. Die Ausgangsverbindungen können in Gegenwart eines inerten, aprotischen Verdünnungs- oder Lösungsmittels umgesetzt werden. Auch das in überschüssiger Menge verwendete Diamin V ist gegebenenfalls als Verdünnungs-oder Lösungsmittel geeignet. Zur Durchführung der Cyclisierung wird anschließend nach Entfernen von überschüssigem Diamin und/oder Lösungsmittel der Rückstand in einem inerten, hochsiedenden Lösungsmittel, vorzugweise Tetralin, auf Temperaturen zwischen 150 und 220 ° C, zweckmäßigerweise 180 bis 200 ° C erhitzt. Das Reaktionsprodukt wird in an sich bekannter Weise durch Abdestillieren des Lösungsmittels aus dem Reaktionsgemisch erhalten. Eine Reinigung erfolgt in üblicher Weise, beispielsweise durch Umkristallisieren aus einem Lösungsmittel, durch Säulenchromatographie oder Überführen in eine Säureadditionsverbindung.

Die Ausgangsverbindungen IV können wie folgt hergestellt werden: Eine entsprechend substituierte o-Aminobenzoesäure der allgemeinen Formel VII

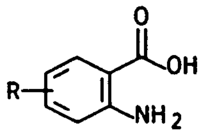

VII,

in der R wie eingangs definiert ist, wird mit einem ω-Halogenketon der Formel III in einem inerten Lösungsmittel, z. B. Benzol, Toluol, Xylol oder Chlor- bzw. Dichlorbenzol, zweckmäßigerweise in Anwesenheit katalytischer Mengen Säure, beispielsweise p-Toluolsulfonsäure, umgesetzt. Die Umsetzung erfolgt zweckmäßigerweise bei der Siedetemperatur des verwendeten Lösungsmittels und unter azeotroper Entfernung des bei der Reaktion gebildeten Wassers. Nach Beendigung der Reaktion können die Produkte in üblicher Weise durch Abdestillieren des Lösungsmittels erhalten und durch Umkristallisieren aus einem geeigneten Lösungsmittel, vorzugsweise einem Alkohol mit 1 bis 4 C-Atomen, gereinigt werden.

Verbindungen der Formel I, in denen A und R die angegebene Bedeutung haben und X eine durch eine Hydroxygruppe substituierte Phenyl- oder Naphthyl-gruppe bedeutet, können nach bekannten Verfahren alkyliert werden. Zur Alkylierung mit den Aminoalkylhalogeniden VI wird beispielsweise aus der Hydroxyverbindung zunächst mit NaH in einem inerten aprotischen Lösungsmittel wie z. B. Dimethylformamid, Dimethylacetamid, Dimethylsulfoxid oder Dimethoxyethan das Anion gebildet und dieses dann mit dem Alkylierungsmittel umgesetzt. Die Bildung des Anions erfolgt bei Temperaturen zwischen 0 und 100 °C, vorzugsweise zwischen 20 und 50 °C. Die Umsetzung des Anions mit dem Alkylierungsmittel erfolgt zwischen 0 und 80 °C, vorzugsweise zwischen Raumtemperatur und 50 °C. Die Umsetzung mit Alkylsulfonylchloriden erfolgt in inerten aprotischen Lösungsmitteln, wie beispielsweise Methylenchlorid oder Chloroform unter Zusatz eines säurebindenden Mittels, vorzugsweise eines tertiären Amins, beispielsweise Pyridin oder Triethylamin. Die Reaktion kann bei Temperaturen zwischen -20 °C und der Siedetemperatur des verwendeten Lösungsmittels, bevorzugt zwischen 0 und 60 °C, insbesondere bei Raumtemperatur, durchgeführt werden. Die Isolierung und Reinigung der Produkte erfolgt nach an sich bekannten Methoden.

Gegebenenfalls werden die erhaltenen erfindungsgemäßen Verbindungen in ihre Säureadditionssalze mit physiologisch verträglichen Säuren überführt. Als übliche physiologisch verträgliche organische oder anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere können aus Fortschritte der Arzneimittelforschung, Bd. 10, S. 224 f., Birkhäuser Verlag, Basel und Stuttgart, 1966 entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem Ether, wie Diethylether oder Methyl-t-butylether, erhalten. Zur besseren Kristallabscheidung können auch Mischungen der genannten Lösungsmittel verwendet werden. Darüber hinaus können pharmazeutisch vertretbare wäßrige Lösungen von Säureadditionsverbindungen der erfindungsgemäßen Verbindung I durch Auflösung der freien Basen in einer wäßrigen Säurelösung hergestellt werden.

Die erfindungsgemäßen Verbindungen der Formel I besitzen ein Chiralitätszentrum und werden als Racemate erhalten, die nach bekannten Methoden, beispielsweise durch Bildung diastereomerer Salze mit optisch aktiven Hilfsäuren, in die optisch aktiven Antipoden getrennt werden können.

In einigen Fällen weisen die erfindungsgemäßen Verbindungen der Formel I je nach Auswahl der verwendeten Amine V ein zweites asymmetrisches Kohlenstoffatom auf und können dann als Diastereomerengemische vorliegen, die in bekannter Weise mit physikalisch-chemischen Methoden in Diastereomerenpaare getrennt werden können.

Die neuen Verbindungen stellen Antiarrhythmika dar. Letztere werden nach Vaughan Williams in vier Gruppen eingeteilt:

- I. Na-Kanalhemmstoffe,
- II. Adrenerge ß-Rezeptorenblocker
- III. K-Kanalhemmstoffe und
- IV. Ca-Antagonisten.

Die neuen Verbindungen sind der Klasse III zuzuordnen. Als solche sind sie in der Therapie anderen Antiarrhythmika vorzuziehen, da sie bei sonst therapieresistenten Arrhythmien verschiedener Genese wirken. Sie beseitigen ventrikuläre Arrhythmien, die nach Herzinfarkt auftreten und auf einem re-entry-Mechanismus beruhen. Weiter wirken sie auch gut bei atrialen Herzrhythmusstörungen. Klasse-III-Antiarrhythmika führen zu Verlängerung der QT-Strecke im EKG, ohne die PQ-Dauer zu beeinflussen und ohne die Herzfrequenz stärker zu senken.

Die Wirkung der neuen Substanzen wurden wie folgt getestet:

Als Versuchstiere dienten männliche und weibliche Pirbright-white-Meerschweinchen im Gewicht von 300-500 g. Die Narkose erfolgte mit 1,5 g/kg Urethan i.p. Die Substanzen wurden intravenös appliziert. Zur Messung der EKG-Leitungszeiten und der Herzfrequenz wurde die II. Extremitätenableitung registriert. Meßgrößen waren die QT- und die PQ-Strecken und die Herzfrequenz. Pro Dosis wurden 4-6 Tiere eingesetzt. Kriterium für Klasse-III-Wirkung war die Zunahme der QT-Dauer im Vergleich zu den Werten vor Substanzgabe. PQ-Zunahme und starke Herzfrequenzabnahme dienten als Ausschlußkriterien. Aus der linearen Beziehung zwischen log Dosis (mg/kg) der Substanz und der relativen Verlängerung der QT-Dauer ($\Delta$%) wird die ED20 % berechnet. Die Tabelle zeigt die erhaltenen Werte sowie die relative Wirkstärke bezogen auf d-Sotalol.

Tabelle

| Substanz des Beispiels Nr. | Verlängerung der QT-Dauer $ED_{20\%}$ mg/kg | Relative Wirkstärke bezogen auf d-Sotalol = 1,0 |
|---|---|---|
| 1 | 1,0 | 3,6 |
| 2 | 1,1 | 3,3 |
| 4 | 0,21 | 17 |
| 5 | 0,86 | 4,3 |
| 6 | 0,61 | 6,0 |
| 7 | 1,5 | 2,4 |
| 9 | 0,40 | 9,0 |
| 11 | 0,99 | 3,7 |
| 13 | 0,43 | 8,5 |
| 17 | 1,4 | 2,6 |
| 19 | 0,46 | 8,0 |
| 20 | 0,56 | 6,5 |
| 22 | 0,29 | 12 |
| 23 | 0,58 | 6,2 |
| 24 | 1,5 | 2,4 |
| 25 | 0,42 | 8,7 |
| 26 | 0,94 | 3,9 |
| 27 | 0,55 | 6,6 |
| 28 | 1,0 | 3,6 |
| 29 | 0,56 | 6,5 |
| 31 | 1,5 | 2,4 |
| 32 | 0,61 | 6,0 |
| 33 | 0,63 | 5,7 |
| 35 | 0,69 | 5,3 |
| 38 | 0,46 | 7,8 |
| 39 | 0,78 | 4,7 |
| 40 | 0,60 | 6,1 |
| 41 | 0,49 | 7,4 |
| 42 | 0,59 | 6,2 |
| 43 | 0,55 | 6,7 |
| 44 | 0,52 | 7,0 |
| 45 | 0,31 | 11,8 |
| 46 | 0,32 | 11 |
| 47 | 0,25 | 15 |
| 49 | 1,0 | 3,6 |
| d-Sotalol | 3,6 | 1,0 |

Wie die Tabelle zeigt, sind die erfindungsgemäßen Substanzen hinsichtlich QT-Verlängerung 2,4- bis 17-fach wirksamer als das bekannte Klasse-III-Antiarrhythmikum d-Sotalol.

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis zwischen 10 und 500 mg pro Patient und Tag bei oraler Gabe und zwischen 1 und 50 mg pro Patient und Tag bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die folgenden Beispiele sollen die Erfindung näher erläutern.

Beispiel 1:

2,3,5,6,7,8-Hexahydro-5-(4-methylphenyl)-imidazo(1,2-c)pyrrolo-(1,2-a)chin azolin

Eine Mischung aus 10,2 g 4-Chlor-1-(4-methylphenyl)-butan-1-on, 8,1 g 2-(2-Aminophenyl)-4,5-dihydroimidazol, 0,5 g Natriumiodid und 250 ml Ethanol wurde bei Raumtemperatur mit 5,5 ml 12 N HCl versetzt und anschließend 30 h unter Rückfluß gekocht. Das Lösungsmittel wurde entfernt und der Rückstand 4 h unter $N_2$-Atmosphäre auf 120 °C erhitzt. Nach dem Abkühlen wurde der Rückstand in $H_2O$/Methyl-t-butylether verteilt und die ungelösten Bestandteile abgesaugt. Die Phasen wurden getrennt und die wäßrige Phase mit 5 N Natronlauge alkalisch gemacht, wobei sich ein gelbbrauner Stoff in Form eines Öls bildete, der durch dreimaliges Ausschütteln mit Methylenchlorid extrahiert wurde. Nach dem Trocknen mit $MgSO_4$ wurde filtriert, das Lösungsmittel abdestilliert und das Rohprodukt (13,1 g) heiß in Essigsäureethylester gelöst. Beim Abkühlen fiel das Produkt in Form farbloser Kristalle aus. Ausbeute: 10,8 g (71 %), Fp. 187-189 °C;

$C_{20}H_{21}N_3$ (303,4)

| | | |
|---|---|---|
| ber. C 79,17 | H 6,98 | N 13,85 |
| gef. C 78,9 H | 7,1 | N 13,8 |

Analog Beispiel 1 wurden hergestellt:

2. 5-(4-Chlorphenyl)-2,3,5,6,7,8-hexahydro-imidazo(1,2-c)pyrrolo(1,2-a)chinazolin
Ausbeute: 7,2 g (36 %), Fp. 158-160 °C (Ethanol)
3. 12-Chlor-2,3,5,6,7,8-hexahydro-5-phenyl-imidazo(1,2-c)pyrrolo1,2-a)chinazolin
Ausbeute: 8,9 g (54 %); Fp. 249-250 °C (Ethanol)
4. 5-(4-Fluorphenyl)-2,3,5,6,7,8-hexahydro-imidazo(1,2-c)pyrrolo(1,2-a)chinazolin
Ausbeute: 10,5 g (68 %); Fp. 177 °C (Zersetzung)
5. 2,3,5,6,7,8-Hexahydro-5-(4-hydroxyphenyl)-imidazo(1,2-c)pyrrolo(1,2-a)chinazolin-hydrochlorid
Ausbeute: 25,3 g (74 %); Fp. 275-277 °C (Zersetzung) (Methanol/Ethanol)
6. 2,3,5,6,7,8-Hexahydro-5-(4-methoxyphenyl)-imidazo(1,2-c)pyrrolo(1,2-a)chinazolin
Ausbeute: 7,7 g (48 %); Fp. 110-112 °C
7. 5-(3,4-Dichlorphenyl)-2,3,5,6,7,8-hexahydro-imidazo(1,2-c)pyrrolo-(1,2 -a)chinazolin
Ausbeute: 4,4 g (71 %); Fp. 218-222 °C
8. 2,3,5,6,7,8-Hexahydro-5-(2,4-dimethylphenyl)-imidazo(1,2-c)-pyrrolo(1,2-a)chinazolin
Ausbeute: 5,0 g (32 %); Fp. 175-180 °C (Zersetzung)
9. 11-Chlor-2,3,5,6,7,8-hexahydro-5-phenyl-imidazo(1,2-c)pyrrolo(1,2-a)chinazolin
Ausbeute: 10,0 g (62 %); Fp. 177-181 °C
10. 2,3,5,6,7,8-Hexahydro-5-(2-thienyl)-imidazo(1,2-c)pyrrolo(1,2-a)-chinazolin Ausbeute: 7,5 g (51 %); Fp. 159-161 °C
11. 5-(2,4-Dichlorphenyl)-2,3,5,6,7,8-hexahydro-imidazo(1,2-c)pyrrolo-(1,2-a)chinazolin-hydrochlorid
Ausbeute: 10,1 g (51 %); Fp. 200 °C (Zersetzung) (Ethanol/Methyl-t-butylether)
12. 5-(4-Bromphenyl)-2,3,5,6,7,8-hexahydro-imidazo(1,2-c)pyrrolo(1,2-a)chinazolin-hydrochlorid
Ausbeute: 8,2 g (52 %); Fp. 273-274 °C (Ethanol/Methyl-t-butylether)

13. 11-Chlor-5-(4-fluorphenyl)-2,3,5,6,7,8-hexahydro-imidazo(1,2-c)pyrrolo(1,2-a)chinazolin-hydrochlorid
Ausbeute: 16,9 g (82 %); Fp. 240-243 ° C (Ethanol/Methyl-t-butylether)
14. 5-(4-Aminophenyl)-2,3,5,6,7,8-hexahydro-imidazo(1,2-c)pyrrolo(1,2-a)chinazolin-hydrochlorid
Ausbeute: 3,9 g (32 %); Fp. 259-261 ° C (Ethanol/Diethylether)
15. 2,3,5,6,7,8-Hexahydro-5-(α-naphtyl)-imidazo(1,2-c)pyrrolo(1,2-a)chinazolin
Ausbeute: 6,4 g (61 %); Fp. 216-217 ° C (Zersetzung)
16. 2,3,5,6,7,8-Hexahydro-5-(3-nitrophenyl)-imidazo(1,2-c)pyrrolo(1,2-a)chinazolin-hydrochlorid
Ausbeute: 8,0 g (86 %); Fp. 297-298 ° C (Ethanol/Methyl-t-butylether)
17. 5-(2-(5-Chlorthienyl))-2,3,5,6,7,8-hexahydro-imidazo(1,2-c)-pyrrolo(1,2-a)chinazolin-hydrochlorid
Ausbeute: 13,4 g (73 %); Fp. 245-248 ° C (Ethanol/Diethylether)
18. 2,3,5,6,7,8-Hexahydro-5-(4-methylsulfonylaminophenyl)-imidazo(1,2-c)pyrrolo(1,2-a)chinazolin-hydrochlorid
Ausbeute: 8,4 g (59 %); Fp. 296-297 ° C (Methanol)
19. 2,3,5,6,7,8-Hexahydro-5-(4-nitrophenyl)-imidazo(1,2-c)pyrrolo(1,2-a)chinazolin-hydrochlorid
Ausbeute: 1,5 g (51 %); Fp. 295 ° C (Zersetzung) (Ethanol)
20. 2,3,5,6,7,8-Hexahydro-2,3-tetramethylen-5-phenyl-imidazo(1,2-c)pyrrol(1,2-a)chinazolin
Ausbeute: 3,9 g (51 %); Fp. 168-170 ° C
21. 2,3,5,6,7,8-Hexahydro-5-(4-fluorphenyl)-2,3-tetramethylenimidazo(1,2-c)pyrrolo(1,2-a)chinazolin-hydrochlorid
Ausbeute: 5,7 g (67 %); Fp. 160 ° C (Zersetzung) (Ethanol)
22. 2,3,5,6,7,8-Hexahydro-3-methyl-5-phenyl-imidazo(1,2-c)pyrrolo(1,2-a)chinazolin-hydrochlorid
Ausbeute: 2,8 g (29 %); Fp. 238-240 ° C (Ethanol/Methyl-t-butylether)
23. 2,3,6,7,8,9-Hexahydro-6-(2-thienyl)-2H-pyrimido(1,2-c)pyrrolo-(1,2-a)chinazolin
Ausbeute: 5,2 g (34 %); Fp. 136-138 ° C
24. 6-(4-Chlorphenyl)-2,3,6,7,8,9-hexahydro-2H-pyrimido(1,2-c)pyrrolo(1,2-a)chinazolin Ausbeute: 10,3 g (61 %); Fp. 148-150 ° C
25. 6-(4-Fluorphenyl)-2,3,5,6,7,8-hexahydro-2H-pyrimido(1,2-c)pyrrolo-(1,2-a,2-a)chinazolin
Ausbeute: 9,1 g (57 %); Fp. 154-156 ° C
26. 6-(3,4-Dichlorphenyl)-2,3,5,6,7,8-hexahydro-2H-pyrimido-(1,2-c)pyrrolo(1,2 -a)chinazolin
Ausbeute: 3,5 g (51 %); Fp. 155-157 ° C
27. 13-Chlor-2,3,5,6,7,8-hexahydro-6-phenyl-2H-pyrimido(1,2-c)pyrrolo(1,2-a)chinazolin-hydrochlorid
Ausbeute: 4,5 g (24 %); Fp. 320 ° C (Zersetzung) (Ethanol/Methyl-t-butylether)
28. 13-Chlor-6-(4-fluorphenyl)-2,3,5,6,7,8-hexahydro-2H-pyrimido(1,2-c)pyrrolo(1,2-a)chinazolin
Ausbeute: 9,3 g (53 %); Fp. 197-201 ° C
29. 2,3,5,6,7,8-Hexahydro-6-(3-nitrophenyl)-2H-pyrimido(1,2-c)pyrrolo(1,2-a)chinazolin
Ausbeute: 7,5 g (47 %); Fp. 141-143 ° C
30. 2,3,5,6,7,8-Hexahydro-6-(4-methylsulfonylaminophenyl)-2H-pyrimido(1,2-c)pyrrolo(1,2-a)chinazolin-hydrochlorid
Ausbeute: 5,6 g (41 %); Fp. 175-177 ° C (Zersetzung) (Aceton/Wasser)
31. 6-(2-(5-Chlorthienyl)-2,3,5,6,7,8-hexahydro-2H-pyrimido(1,2-c)pyrrolo(1,2-a)chinazolin
Ausbeute: 5,6 g (33 %); Fp. 245 ° C (Zersetzung) (Ethanol/Diethylether)
32. 2,3,5,6,7,8-Hexahydro-6-(4-nitrophenyl)-2H-pyrimido(1,2-c)pyrrolo(1,2-a)chinazolin
Ausbeute: 9,6 g (61 %); Fp. 287 ° C (Zersetzung) (Methanol)
33. 2,3,5,6,7,8-Hexahydro-6-(4-methoxyphenyl)-2H-pyrimido(1,2-c)pyrrolo(1,2-a)chinazolin-hydrochlorid
Ausbeute: 4,1 g (22 %); Fp. 247 ° C (Ethanol/Methyl-t-butylether)
34. 6-(2,4-Dichlorphenyl)-2,3,5,6,7,8-hexahydro-2H-pyrimido(1,2-c)pyrrolo(1,2-a)chinazolin-fumarat
Ausbeute: 6,0 g (40 %); Fp. 207-209 ° C (Zersetzung) (Ethanol/Methyl-t-butylether)
35. 12-Chlor-2,3,5,6,7,8-hexahydro-6-phenyl-2H-pyrimido(1,2-c)pyrrolo(1,2-a)chinazolin-hydrochlorid
Ausbeute: 10,8 g (58 %); Fp. 289-292 ° C (Ethanol)
36. 2,3,5,6,7,8-Hexahydro-6-(4-hydroxyphenyl)-2H-pyrimido(1,2-c)pyrrolo(1,2-a)chinazolin-hydrochlorid
Ausbeute: 11,0 g (62 %); Fp. 150 ° C (Zersetzung) (Ethanol)
37. 6-(4-Bromphenyl)-2,3,5,6,7,8-hexahydro-2H-pyrimido(1,2-c)pyrrolo(1,2-a)chinazolin-hydrochlorid
Ausbeute: 7,0 g (43 %); Fp. 298-302 ° C (Zersetzung) (Ethanol/Methyl-t-butylether)
38. 2,3,5,6,7,8-Hexahydro-3,3-dimethyl-6-phenyl-2H-pyrimido(1,2-c) pyrrolo(1,2-a)chinazolin-hydrochlorid
Ausbeute: 8,9 (49 %); Fp. 304-306 ° C (Ethanol)
39. 2,3,5,6,7,8-Hexahydro-3,3-dimethyl-6-(4-nitrophenyl)-2H-pyrimido(1,2-c)-pyrrolo(1,2-a)chinarolin-pyrdochlorid
Ausbeute: 2,6 g(63 %); Fp. 326 ° C (Ethanol)

40. 2,3,5,6,7,8-Hexahydro-3,3-dimethyl-5-phenyl-imidazo(1,2c-)pyrrolo(1,2 -a)chinazolin-hydrochlorid
Ausbeute: 15,0 g (80 %); Fp 269 ° C (Ethanol)
41. 2,3,5,6,7,8-Hexahydro-4,4-dimethyl-6-phenyl-2H-pyrimido(1,2-c)pyrrolo(1,2-a)chinazolin-hydrochlorid
Ausbeute: 6,5 g (72 %); Fp. 287 ° C (Ethanol/Methyl-t-butylether)
42. 5-(4-Fluorphenyl)-2,3,5,6,7,8-hexahydro-3,3-dimethyl-imidazo(1,2-c-)pyrrolo-(1,2-a)chinazolin-hydrochlorid
Ausbeute: 3,9 g (35 %), Fp. 251 ° C (Ethanol)
43. 2,3,5,6,7,8-Hexahydro-3,3-dimethyl-5-(4-nitrophenyl)-imidazo(1,2-c)pyrrolo-(1,2-a)chinazolin-hydrochlorid
Ausbeute: 5,3 g (46 %); Fp >300 ° C (Ethanol)
44. 2,3,5,6,7,8-Hexahydro-4,4-dimethyl-6-(4-nitrophenyl)-2H-pyrimido(1,2-c)-pyrrolo(1,2-a) chinazolin-hydrochlorid
Ausbeute: 5,0 g (47 %); Fp. >300 ° C (Ethanol)
45. 2,3,5,6,7,8-Hexahydro-3-methyl-5-(4-nitrophenyl)-imidazo(1,2-c)pyrrolo-(1,2-a)chinazolin-hydrochlorid
Ausbeute: 5,1 g (46 %); Fp. 283-285 ° C (Ethanol)

Beispiel 46

2,3,5,6,7,8-Hexahydro-6-phenyl-2H-pyrimido(1,2-c)pyrrolo(1,2-a)chinazolin-hydrochlorid

a) 1,2,3,3a-Tetrahydro-3a-phenyl-5H-pyrrolo(1,2-a)(3,1)benzoxazin-5-on (Formel IV, $R^3$ = H, X = $C_6H_5$)

Eine Mischung von 26,1 g 2-Aminobenzoesäure, 36,5 g 4-Chlor-1-phenyl--butan-1-on, 0,5 g p-Toluolsulfonsäure und 250 ml Xylol wurde am Wasserabscheider zum Rückfluß erwärmt. Nach Beendigung der Reaktion wurde das Lösungsmittel abdestilliert und der zurückbleibende Feststoff aus Ethanol umkristallisiert. Ausbeute: 41,1 g (81,5 %), Fp. 152 ° C

b) 2,3,5,6,7,8-Hexahydro-6-phenyl-2H-pyrimido(1,2-c)pyrrolo(1,2-a)-chinazolin-hydrochlorid

6,2 g 1,2,3,3a-Tetrahydro-3a-phenyl-5H-pyrrolo(1,2-a)(3,1)benzoxazin-5-on wurden in 15 ml 1,3-Diaminopropan gelöst und 3 h bei 80 ° C gerührt. Zu dieser Lösung wurden anschließend 35 ml Tetralin gegeben, die Innentemperatur innerhalb von 30 min auf 190 ° C gesteigert und das überschüssige Diamin abdestilliert. Nach 6 h wurde im Vakuum bei 190 ° C eingeengt, der Rückstand auf etwa 70 ° C abkühlen gelassen und in 15 ml Ethanol gelöst. Das nach Zusatz von 8 ml 3 N etherischer HCl langsam auskristallisierende Rohprodukt wurde abgesaugt und mit wenig Ethanol gewaschen.
Ausbeute: 4,4 g (55,5 %); Fp. 285-286 ° C

Analog Beispiel 46 wurde hergestellt:

47. 2,3,5,6,7,8-Hexahydro-5-phenyl-imidazo(1,2-c)pyrrolo(1,2-a)-chinazolin-hydrochlorid
Ausbeute: 3,6 g (45 %); Fp. 308-310 ° C

Beispiel 48

2,3,5,6,7,8-Hexahydro-5-(4-(2-dimethylaminoethoxy)-phenyl)-imidazo(1,2-c)pyrrolo(1,2-a)chinazolin-hyrochlorid

5 g 2,3,5,6,7,8-Hexahydro-5-(4-hydroxyphenyl)-imidazo-(1,2-c)pyrrolo(1,2-a)chinazolin-hydrochlorid (Beispiel 7), suspendiert in 200 ml Dimethylformamid, wurden langsam bei Raumtemperatur zu einer Suspension von 1,5 g Natriumhydrid in 100 ml Dimethylformamid gegeben. Nach Beendigung der Gasentwicklung wurde mit 3 g 2-Chlor-N,N-dimethylethylamin versetzt und 20 h bei Raumtemperatur gerührt. Die Reaktionslösung wurde mit $H_2O$ versetzt und mit Methylenchlorid extrahiert. Die organische Phase wurde mit $H_2O$ gewaschen, über Magnesiumsulfat getrocknet und eingeengt. Der verbleibende ölige Rückstand wurde in wenig Isopropanol aufgenommen, mit 4,5 ml 3 N etherischer HCl versetzt und der sich abscheidende Feststoff aus Ethanol/Methyl-t-butylether umkristallisiert. Ausbeute: 2,0 g (33 %); Fp. 304 ° C (Zersetzung).

Analog wurde hergestellt:

49. 2,3,5,6,7,8-Hexahydro-5-(4-hydroxyphenyl)-imidazo(1,2-c)pyrrolo-(1,2-a)chinazolin-methylsulfonsäureester-hydrochlorid

Zu einer Suspension von 8,55 g 2,3,5,6,7,8-Hexahydro-5-(4-hydroxyphenyl)-imidazo(1,2-c)pyrrolo-(1,2-a)chinazolin-hydrochlorid in 100 ml Methylenchlorid und 25 ml Pyridin wurden bei 0-5°C unter gutem Rühren 6 ml Methylsulfonylchlorid zugetropft und die Reaktionsmischung 70 h bei Raumtemperatur gerührt. Nach Abdestillieren des Lösungsmittels wurde mit 1 N HCl versetzt und der entstandene Niederschlag aus Ethanol/Methyl-t-butylether umkristallisiert. Ausbeute: 8,0 g (76 %); Fp. 241-242°C (Zersetzung).

Beispiele für galenische Applikationsformen:

A) Auf einer Tablettenpresse werden in üblicher Weise Tabletten folgender Zusammensetzung gepreßt:
40 mg Substanz des Beispiels 26
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6 %iger Kleister)
B) 20 mg Substanz des Beispiels 39
60 mg Kernmasse
60 mg Verzuckerungsmasse
Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.
C) 10 g Substanz des Beispiels 38 werden in 5000 ml Wasser unter Zusatz von NaCl gelöst und mit 0,1 N NaOH auf pH 6,0 eingestellt, so daß eine blutisotonische Lösung entsteht. Jeweils 1 ml dieser Lösung werden in Ampullen gefüllt und sterilisiert.

## Patentansprüche

## Patentansprüche für folgende Vertragsstaaten : AT, BE, CH, DE, FR, GB, IT, LI, NL

**1.** Tetracyclische Chinazolinderivate der Formel I

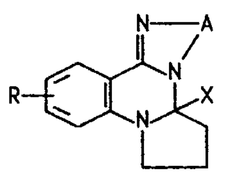

I,

worin

- A eine gegebenenfalls $C_{1-4}$-alkylsubstituierte $C_{2-4}$-Alkylengruppe, worin 2 benachbarte Methylengruppen zusätzlich über eine gegebenenfalls $C_{1-4}$-alkylsubstituierte $C_3$-$C_5$-Alkylengruppe zu einem Ring geschlossen sein können,
- X einen gegebenenfalls durch Halogen, Nitro, Amino, $C_{1-4}$-Alkylamino, Sulfonylamino, $C_{1-4}$-Acylamino, Hydroxy, $C_{1-4}$-Alkoxy, $-O(CH_2)_{2-4}-NR^1R^2$ (mit $R^1$ und $R^2$ in der Bedeutung von Wasserstoff oder $C_{1-4}$-Alkyl), $C_{1-4}$-Alkyl oder eine $C_{1-4}$-Alkylsulfonsäuregruppe substituierten Phenyl-oder Naphthylring oder einen gegegebenenfalls durch Chlor, Fluor, Methyl, Ethyl, Methoxy, Ethoxy, Nitro oder Hydroxy substituierten Thiophenring und
- R Wasserstoff, Halogen oder $C_{1-4}$-Alkoxy

bedeuten, sowie deren Salze mit physiologisch verträglichen Säuren.

**2.** Tetracyclische Chinazolinderivate der Formel I gemäß Anspruch 1, dadurch gekennzeichnet, daß

- A eine gegebenenfalls durch ein oder zwei $C_{1-4}$-Alkylreste substituierte $C_{2-3}$-Alkylengruppe,
- X einen durch Wasserstoff, Chlor, Fluor, Methyl, Ethyl, Methoxy, Ethoxy, Nitro oder Hydroxy substituierten Phenyl oder Thiophenring und
- R Wasserstoff, Fluor, Chlor, Methoxy und Ethoxy

bedeuten.

**3.** Tetracyclische Chinazolinderivate gemäß Anspruch 2, dadurch gekennzeichnet, daß A einen durch ein oder zwei Methylreste substituierten $C_{2-3}$-Alkylenrest bedeutet.

**4.** Verfahren zur Herstellung der tetracyclischen Chinazolinderivate der Formel I gemäß Anspruch 1, dadurch gekennzeichnet, daß man

a) eine Verbindung der Formel II

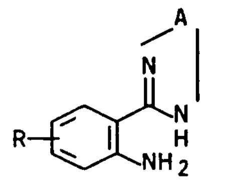

II,

in der R und A die angegebenen Bedeutungen haben, mit einem ω-Halogenketon der Formel III

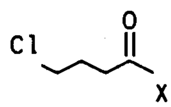

III,

in dem X die angegebene Bedeutung hat, umsetzt oder

b) eine Verbindung der Formel IV

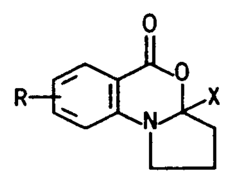

IV,

worin R und X die angegebenen Bedeutungen besitzen, mit einem Diamin der Formel V

$H_2N\text{-}A\text{-}NH_2$ V ,

in der A die angegebene Bedeutung hat, umsetz

und die so erhaltenen Verbindungen der Formel I, falls X eine durch eine Hydroxygruppe substituierte Phenyl- oder Naphthyl-gruppe bedeutet, gegebenenfalls mit einem Alkylhalogenid der Formel VI

$Hal\text{-}(CH_2)_{2-4}\text{-}NR^1R^2$ VI ,

worin Hal ein Halogenatom bedeutet und $R^1$ und $R^2$ die angegebenen Bedeutungen haben, oder einem $C_{1-4}$-Alkylsulfonylchlorid umsetzt und anschließend die erhaltenen Verbindungen gegebenenfalls in ihre Salze mit physiologisch verträglichen Säuren überführt.

**5.** Tetracyclische Chinazolinderivate der Formel I gemäß Anspruch 1 zur Verwendung bei der Bekämpfung von Krankheiten.

**6.** Verwendung der tetracyclischen Chinazolinderivate der Formel I gemäß Anspruch 1 zur Herstellung von Arzneimitteln zur Bekämpfung von atrialen und ventrikulären Herzrhythmusstörungen.

**Patentansprüche für folgenden Vertragsstaat : ES**

**1.** Verfahren zur Herstellung tetracyclischer Chinazolinderivate der Formel I

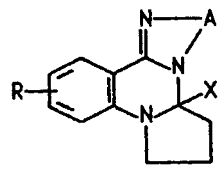

I,

worin

A eine gegebenenfalls $C_{1-4}$-alkylsubstituierte $C_{2-4}$-Alkylengruppe, worin 2 benachbarte Methylengruppen zusätzlich über eine gegebenenfalls $C_{1-4}$-alkylsubstituierte $C_3$-$C_5$-Alkylengruppe zu einem Ring geschlossen sein können,

X einen gegebenenfalls durch Halogen, Nitro, Amino, $C_{1-4}$-Alkylamino, Sulfonylamino, $C_{1-4}$-Acylamino, Hydroxy, $C_{1-4}$-Alkoxy, $-O(CH_2)_{2-4}-NR^1R^2$ (mit $R^1$ und $R^2$ in der Bedeutung von Wasserstoff oder $C_{1-4}$-Alkyl), $C_{1-4}$-Alkyl oder eine $C_{1-4}$-Alkylsulfonsäuregruppe substituierten Phenyl-oder Naphthylring oder einen gegebenenfalls durch Chlor, Fluor, Methyl, Ethyl, Methoxy, Ethoxy, Nitro oder Hydroxy substituierten Thiophenring und

R Wasserstoff, Halogen oder $C_{1-4}$-Alkoxy

bedeuten, sowie deren Salze mit physiologisch verträglichen Säuren, dadurch gekennzeichnet, daß man

a) eine Verbindung der Formel II

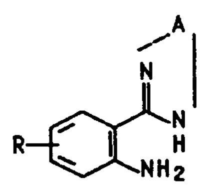

II,

in der R und A die angegebenen Bedeutungen haben, mit einem ω-Halogenketon der Formel III

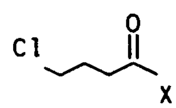

III,

in dem X die angegebene Bedeutung hat, umsetzt oder

b) eine Verbindung der Formel IV

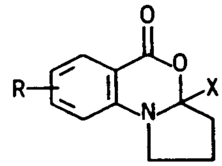

IV,

worin R und X die angegebenen Bedeutungen besitzen, mit einem Diamin der Formel V

$H_2N-A-NH_2$ V ,

in der A die angegebene Bedeutung hat, umsetzt

und die so erhaltenen Verbindungen der Formel I, falls X eine durch eine Hydroxygruppe substituierte Phenyl- oder Naphthylgruppe bedeutet, gegebenenfalls mit einem Alkylhalogenid der Formel VI

$Hal-(CH_2)_{2-4}-NR^1R^2$ VI ,

worin Hal ein Halogenatom bedeutet und $R^1$ und $R^2$ die angegebenen Bedeutungen haben, oder einem $C_{1-4}$-Alkylsulfonylchloride umsetzt und anschließend die erhalten Verbindungen gegebenenfalls in ihre Salze mit physiologisch Säuren überführt.

**2.** Verfahren gemäß Anspruch 1, dadurch gekennzeichnet, daß man Chinazolinderivate der Formel I herstellt, in denen

A eine gegebenenfalls durch ein oder zwei $C_{1-4}$-Alkylreste substituierte $C_{2-3}$-Alkylengruppe,

X einen durch Wasserstoff, Chlor, Fluor, Methyl, Ethyl, Methoxy, Ethoxy, Nitro oder Hydroxy substituierten Phenyl oder Thiophenring und

R Wasserstoff, Fluor, Chlor, Methoxy und Ethoxy

bedeuten.

**3.** Verfahren gemäß Anspruch 1, dadurch gekennzeichnet, daß man Chinazolinderivate der Formel I herstellt, in denen A einen durch ein oder zwei Methylreste substituierten $C_{2-3}$-Alkylenrest bedeutet.

**Claims**

**Claims for the following Contracting States : AT, BE, CH, DE, FR, GB, IT, LI, NL**

**1.** A tetracyclic quinazoline derivative of the formula 1

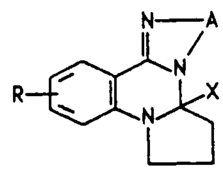

I

where A is $C_{2-4}$-alkylene which may be substituted by $C_{1-4}$-alkyl and in which 2 adjacent methylene groups may additionally be closed to a ring via a $C_3$-$C_5$-alkylene group which may be substituted by $C_{1-4}$-alkyl, x is a phenyl or naphthyl ring which may be substituted by halogen, nitro, amino, $C_{1-4}$-alkylamino, sulfonylamino, $C_{1-4}$-acylamino, hydroxyl, $C_{1-4}$-alkoxy, $-O(CH_2)_{2-4}-NR^1R^2$ (with $R^1$ and $R^2$ meaning hydrogen or $C_{1-4}$-alkyl), $C_{1-4}$-alkyl or $C_{1-4}$-alkylsulfonyl, or is a thiophene ring which may be substituted by chlorine, fluorine, methyl, ethyl, methoxy, ethoxy, nitro or hydroxyl, and R is hydrogen, halogen or $C_{1-4}$-alkoxy, and the salts thereof with physiologically tolerated acids.

**2.** A tetracyclic quinazoline derivative of the formula I as claimed in claim 1, wherein A is $C_{2-3}$-alkylene which may be substituted by one or two $C_{1-4}$-alkyl radicals, X is a phenyl or thienyl ring which is substituted by hydrogen, chlorine, fluorine, methyl, ethyl, methoxy, ethoxy, nitro or hydroxyl, and R is hydrogen, fluorine, chlorine, methoxy or ethoxy.

**3.** A tetracyclic quinazoline derivative as claimed in claim 2, wherein A is $C_{2-3}$-allcylene which is substituted by one or two methyl radicals.

**4.** A process for the preparation of a tetracyclic quinazoline derivative of the formula I as claimed in claim I, which comprises

a) reacting a compound of the formula II

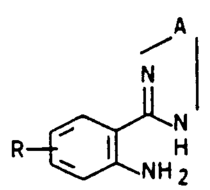

II

where R and A have the stated meanings, with an ω-halogenoketone of the formula III

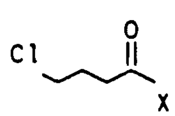

III

where X has the stated meaning, or

b) reacting a compound of the formula IV

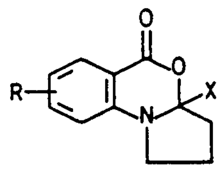

IV

where R and x have the stated meanings, with a diamine of the formula V

$H_2N\text{-}A\text{-}NH_2$ V

where A has the stated meaning,

and possibly reacting the resulting compound of the formula I, if x is a phenyl or naphthyl which is substituted by hydroxyl, with an alkyl halide of the formula VI

$Hal\text{-}(CH_2)_{2-4}\text{-}NR^1R^2$ VI

where Hal is halogen, and R' and $R^2$ have the stated meanings, or with a $C_{1-4}$-alkylsulfonyl chloride, and subsequently possibly converting the resulting compound into a salt thereof with a physiologically tolerated acid.

**5.** A tetracyclic quinazoline derivative of the formula I ad claimed in claim 1 for use for controlling diseases.

**6.** The use of a tetracyclic quinazoline derivative of the formula I as claimed in claim 1 for preparing drugs for controlling atrial and ventricular dysrhythmias.

**Claims for the following Contracting State : ES**

**1.** A process for the preparation of a tetracyclic quinazoline derivative of the formula I

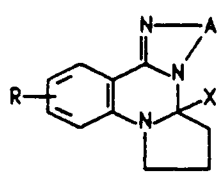

I

where A is $C_{2-4}$-alkylene which may be substituted by $C_{1-4}$-alkyl and in which 2 adjacent methylene groups may additionally be closed to a ring via a $C_3$-$C_5$-alkylene group which may be substituted by $C_{1-4}$-alkyl, X is a phenyl or naphthyl ring which may be substituted by halogen, nitro, amino, $C_{1-4}$-alkylamino, sulfonylamino, $C_{1-4}$-acylamino, hydroxyl, $C_{1-4}$-alkoxy, $-O(CH_2)_{2-4}-NR^1R^2$ (with $R^1$ and $R^2$ meaning hydrogen or $C_{1-4}$-alkyl, $C_{1-4}$-alkyl or $C_{1-4}$-alkylsulfonyl, or is a thiophene ring which may be substituted by chlorine, fluorine, methyl, ethyl, methoxy, ethoxy, nitro or hydroxyl, and R is hydrogen, halogen or $C_{1-4}$-alkoxy, and the salts thereof with physiologically tolerated acids, which comprises

a) reacting a compound of the formula II

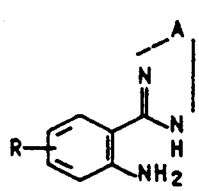

II

where R and A have the stated meanings, with a $\omega$-halogenoketone of the formula III

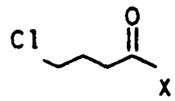

III

where X has the stated meaning, or
b) reacting a compound of the formula IV

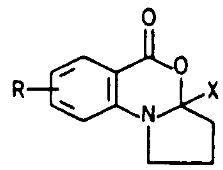

IV

where R and X have the stated meanings, with a diamine of the formula V

$H_2N\text{-}A\text{-}NH_2$ V

where A has the stated meaning,
and possibly reacting the resulting compound of the formula I, if X is a phenyl or naphthyl which is substituted by hydroxyl, with an alkyl halide of the formula VI

$Hal\text{-}(CH_2)_{2-4}\text{-}NR^1R^2$ VI

where Hal is halogen, and $R^1$ and $R^2$ have the stated meanings, or with a $C_{1-4}$-alkylsulfonyl chloride, and subsequently possibly covering the resulting compound into a salt thereof with a physiologically tolerated acid.

**2.** A process as claimed in claim 1, wherein a quinazoline derivative of the formula I is prepared, in which A is $C_{2-3}$-alkylene which may be substituted by one or two $C_{1-4}$-alkyl radicals, X is a phenyl or thienyl ring which is substituted by hydrogen, chlorine, fluorine, methyl, ethyl, methoxy, ethoxy, nitro or hydroxyl, and R is hydrogen, fluorine, chlorine, methoxy or ethoxy.

**3.** A process as claimed in claim 1, wherein a quinazoline derivative of the formula I is prepared, in which A is $C_{2-3}$-alkylene which is substituted by one or two methyl radicals.

**Revendications**
**Revendications pour les Etats contractants suivants : AT, BE, CH, DE, FR, GB, IT, LI, NL**

**1.** Dérivés tétracycliques de la quinazoline, de formule I

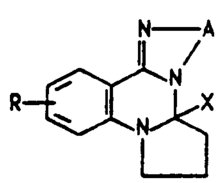

I,

dans laquelle
A représente un groupe alkylène en C 2-C 4 éventuellement substitué par des groupes alkyle en C 1-C 4 et dans lequel deux groupes méthylène voisins peuvent en outre être refermés en un cycle par un groupe alkylène en C 3-C 5 éventuellement substitués par des groupes alkyle en C 1-C 4,
X représente un cycle phényle ou naphtyle éventuellement substitué par des halogènes, des groupes

nitro, amino, alkylamino en C 1-C 4, sulfonylamino, acrylamino en C 1-C 4, hydroxy, alcoxy en C 1-C 4, $-O(CH_2)_{2-4}-NR^1R^2$ (dans lequel $R_1$ et $R_2$ représentant l'hydrogène ou des groupes alkyle en C 1-C 4), alkyle en C 1-C 4 ou un groupe acide (alkyle en C 1-C 4)-sulfonique, ou un cycle thiophène éventuellement substitué par le chlore, le fluor, des groupes méthyle, éthyle, méthoxy, éthoxy, nitro ou hydroxy, et
R représente l'hydrogène, un halogène ou un groupe alcoxy en C 1-C 4,
et leurs sels d'acides acceptables pour l'usage pharmaceutique.

**2.** Dérivés tétracycliques de la quinazoline de formule I de la revendication 1, caractérises en ce que
A représente un groupe alkylène en C 2-C 3 éventuellement substitué par un ou deux groupes alkyle en C 1-C 4,
X représente un cycle phényle ou thiophène substitué par l'hydrogène, le chlore, le fluor, des groupes méthyle, éthyle, méthoxy, éthoxy, nitro ou hydroxy, et
R représente l'hydrogène, le fluor, le chlore, un groupe méthoxy ou éthoxy.

**3.** Dérivés tétracycliques de la quinazoline selon la revendication 2, caractérisés en ce que A représente un groupe alkylène en C 2-C 3 substitué par un ou deux groupes méthyle.

**4.** Procédé de préparation des dérivés tétracycliques de la quinazoline de formule I de la revendication 1, caractérisé en ce que :
a) on fait réagir un composé de formule II

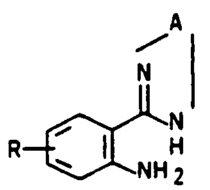

II,

dans laquelle R et A ont les significations indiquées ci-dessus, avec une oméga-halogénocétone de formule III

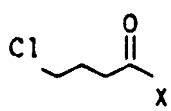

III,

dans laquelle X a les significations indiquées ci-dessus, ou bien
b) on fait réagir un composé de formule IV

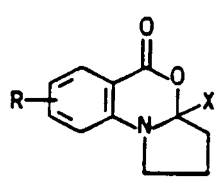

IV,

dans laquelle R et X ont les significations indiquées ci-dessus, avec une diamine de formule V

$H_2N-A-NH_2$ (V)

dans laquelle A a les significations indiquées ci-dessus,
et le cas échéant, lorsque X représente un groupe phényle ou naphtyle substitué par un groupe hydroxy, on fait réagir les composés obtenus, répondant à la formule I, avec un halogènure d'alkyle de formule VI

$Hal-(CH_2)_{2-4}-NR^1R^2$ VI,

dans laquelle Hal représente un atome d'halogène et $R^1$ et $R^2$ ont les significations indiquées ci-dessus, ou avec un chlorure d'(alkyle en C 1-C 4)-sulfonyle puis, le cas échéant, on convertit les composés obtenus en leurs sels d'acides acceptables pour l'usage pharmaceutique.

**5.** Dérivés tétracycliques de la quinazoline de formule I de la revendication 1, pour l'utilisation dans la lutte contre les maladies.

**6.** Utilisation des dérivés tétracycliques de la quinazoline de formule 1 de la revendication 1, pour la préparation de médicaments pour le traitement des troubles auriculaires et ventriculaires du rythme cardiaque.

**Revendications pour l'Etat contractant suivant : ES**

**1.** Procédé de préparation des dérivés tétracycliques de la quinazoline de formule I

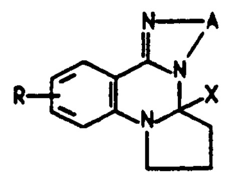

I,

dans laquelle
A représente un groupe alkylène en C 2-C 4 éventuellement substitué par des groupes alkyle en C 1-C 4 et dans lequel deux groupes méthylène voisins peuvent en outre être refermés en un cycle par un groupe alkylène en C 3-C 5 éventuellement substitué par des groupes alkyle en C 1-C 4,
X représente un cycle phényle ou naphtyle éventuellement substitué par des halogènes, des groupes nitro, amino, alkylamino en C 1-C 4, sulfonylamino, acylamino en C 1-C 4, hydroxy, alcoxy en C 1-C 4, $-O(CH_2)_{2-4}-NR^1R^2$ (dans lequel $R_1$ et $R_2$ représentent l'hydrogène ou des groupes alkyle en C 1-C 4), alkyle en C 1-C 4 ou un groupe acide (alkyle en C 1-C 4)-sulfonique, un cycle thiophène éventuellement substitué par le chlore, le fluor, des groupes méthyle, éthyle, méthoxy, éthoxy, nitro ou hydroxy,
R représente l'hydrogène, un halogène ou un groupe alcoxy en C 1-C 4,
et de leurs sels d'acides acceptables pour l'usage pharmaceutique, caractérisé en ce que :

a) on fait réagir un composé de formule II

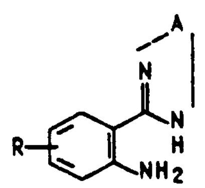

II,

dans laquelle R et A ont les significations indiqués ci-dessus, avec une omégahalogénocétone de formule III

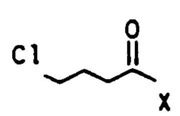

III,

dans laquelle X a les significations indiquées ci-dessus, ou bien

b) on fait réagir un composé de formule IV

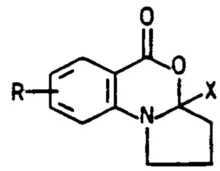

IV,

dans laquelle R et X ont les significations indiquées ci-dessus, avec une diamine de formule V

$H_2N\text{-}A\text{-}NH_2$ (V)

dans laquelle A a les significations indiquées ci-dessus,
et lorsque X représente un groupe phényle ou naphtyle substitué par un groupe hydroxy, on fait éventuellement réagir les composés obtenus, répondant à la formule I, avec un halogénure d'alkyle de formule VI

$Hal\text{-}(CH_2)_{2-4}\text{-}NR^1R^2$ VI,

dans laquelle Hal représente un atome d'halogène et $R^1$ et $R^2$ ont les significations indiquées ci-dessus, ou avec un chlorure d'(alkyle en C 1-C 4)-sulfonyle puis, le cas échéant, on convertit les composés obtenus en leurs sels d'acides acceptables pour l'usage pharmaceutique.

**2.** Procédé selon la revendication 1, caractérisé en ce que l'on prépare des dérivés de la quinazoline de formule I dans laquelle
A représente un groupe alkylène en C 2-C 3 éventuellement substitué par un ou deux groupes alkyle en C 1-C 4,
X représente un cycle phényle ou thiophène substitué par l'hydrogène, le chlore, le fluor, des groupes méthyle, éthyle, méthoxy, éthoxy, nitro ou hydroxy, et
R représente l'hydrogène, le fluor, le chlore, un groupe méthoxy ou éthoxy.

**3.** Procédé selon la revendication 1, caractérisé en ce que l'on prépare des dérivés de la quinazoline de formule I dans laquelle A représente un groupe alkylène en C 2-C 3 substitué par un ou deux groupes méthyle.